(19) Europäisches Patentamt
European Patent Office
Office européen des brevets

(11) Veröffentlichungsnummer: **0 090 238**
**B1**

(12) # EUROPÄISCHE PATENTSCHRIFT

(45) Veröffentlichungstag der Patentschrift: **26.11.86**

(51) Int. Cl.⁴: **C 07 D 239/06**, C 07 D 239/70, C 08 G 59/50

(21) Anmeldenummer: **83102476.5**

(22) Anmeldetag: **14.03.83**

(54) **Cyclische Amidine, Verfahren zu ihrer Herstellung und die Verwendung cyclischer Amidine als Katalysatoren für die Härtung von Epoxidharzen.**

(30) Priorität: **26.03.82 DE 3211301**

(43) Veröffentlichungstag der Anmeldung: **05.10.83 Patentblatt 83/40**

(45) Bekanntmachung des Hinweises auf die Patenterteilung: **26.11.86 Patentblatt 86/48**

(84) Benannte Vertragsstaaten: **AT BE CH DE FR GB IT LI NL SE**

(56) Entgegenhaltungen:
**EP-A-0 000 418**
**EP-A-0 051 787**
**DE-A-1 770 009**
**DE-A-2 132 113**
**DE-A-2 321 054**
**DE-B-1 015 805**
**FR-A-1 350 671**
**FR-A-2 162 416**
**FR-M-7 991**
**GB-A-1 230 347**
**US-A-2 893 993**
**US-A-4 003 718**
**US-A-4 044 009**
**US-A-4 138 563**

**CHEMICAL ABSTRACTS, vol. 82, 1975, Seite 59, Nr. 58863t, Columbus, Ohio, USA**

(73) Patentinhaber: **BAYER AG, Konzernverwaltung RP Patentabteilung, D-5090 Leverkusen 1 Bayerwerk (DE)**

(72) Erfinder: **Meyer, Rolf- Volker, Dr., Buchheimer Strasse 23, D-4150 Krefeld (DE)**
Erfinder: **Kreuder, Hans- Joachim, Dr., Doerperhofstrasse 35, D-4150 Krefeld (DE)**
Erfinder: **Hohl, Erwin, Dipl.- Ing., Kreuzbergstrasse 49, D-4150 Krefeld (DE)**

**EP 0 090 238 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue cyclische Amidine, die neben der -N=C-N- -Funktion noch polare Gruppen enthalten, ein Verfahren zur Herstellung dieser Amidine und die Verwendung cyclischer Amidine als Katalysatoren für die Härtung von Epoxidharzen, d.h. von wärmehärtbaren Massen auf Basis von polyepoxiden und gegebenenfalls polymeren, die Carboxylgruppen enthalten.

Pulverförmige Überzugsmittel, die durch Wirbelsintern, durch Flammspritzen oder nach dem elektrostatischen Pulversprühverfahren auf ein Substrat aufgebracht werden können, sind bekannt. Sie sollen bei mäßig hohen Temperaturen innerhalb möglichst kurzer Zeit zu vernetzten Beschichtungen eingebrannt werden können. Dabei ist zu beachten, daß einerseits beim Extrudieren der Mischung aus Bindemittel, Pigmenten, Füllstoffen und gegebenenfalls weiteren Hilfsmitteln bei Temperaturen von 80 bis 160°C, vorzugsweise 90 bis 120°C, keine Reaktion zwischen Harz und Härter stattfinden darf, andererseits aber ein Bedürfnis nach immer kürzeren Einbrennzeiten bei niedrigen Temperaturen, also nach erhöhter Reaktivität besteht.

Es ist zwar möglich, die Harzkomponente mit einer derart hohen Anzahl von Carboxylgruppen zu versehen, daß bei Einbrennbedingungen von 150-160°C/30 Minuten eine vollständige Reaktion erzielt wird; allerdings tritt aufgrund der hohen Säurezahl schon während des Extrusionsvorgangs eine unerwünschte Vorreaktion ein, die sich nur durch energisches Abkühlen des Extrudats in tolerierbaren Grenzen halten läßt. Auch die Lagerfähigkeit wird dadurch beeinträchtigt, daß selbst bei Raumtemperatur eine unerwünschte Reaktion abläuft, die dann den Verlauf des Pulverlacks und/oder die mechanischen Eigenschaften der Einbrennlackierung negativ beeinflußt.

Bekanntlich kann man die Härtung von carboxylgruppenhaltigen Polymeren mit Polyepoxiden durch Katalysatoren, wie Dicyandiamide oder Stickstoffheterocyclen, wie z.B. Imidazoline (DE-08 22 48 776) oder Tetrahydropyrimidine (DE-08 27 51 805), beschleunigen.

Die EP-A 418 betrifft in der Wärme härtbare Einkomponenten-Klebstoffe auf Basis von Epoxiden, die Imidszolinderivate als Härter enthalten. Carboxylgruppen-haltige Polymere, die sls Reaktionspartner für die Epoxide dienen könnten, werden in der EP-A 418 nicht erwähnt. Es war deshalb durch diese Literaturstelle auch nicht nahegelegt, daß bestimmte cyclische Amidine, die auch Imidazolinderivate umfassen, die Reaktion von Epoxid- mit Carboxylgruppen beschleunigen können.

Die in Chem. Abstracts 82, 58863 t (1975) referierte JP-A 74 14558 betrifft die Verwendung eines substituierten Imidazols als Härter für ein Epoxidharz. Für diese Literaturstelle gilt das oben Gesagte analog.

Neben ausreichender Reaktivität und Lagerstabilität sollen Epoxidharz-Beschichtungen auch eine gute Haltung, insbesondere auf metallischen Untergründen, besitzen. Besonders Diglycidylether des Bisphenols A mit einem Schmelzpunkt von 50 bis 120°C und einem Epoxidäquivalentgewicht von 400 bis 2000 bereiten in dieser Hinsicht Schwierigkeiten. In der Praxis konkurrenzfähig werden Überzugsmittel aber erst dann, wenn sie nicht nur reaktiv und lagerstabil sind und zu haftfesten Überzügen führen, sondern wenn die daraus hergestellten Beschichtungen darüber hinaus auch hochglänzend, elastisch und lösungsmittelbeständig sind.

Es wurde nun gefunden, daß sämtliche oben aufgezählten wünschenswerten Eigenschaften begünstigt werden, wenn man die als Katalysatoren bekannten Amidine durch Einbau zusätzlicher polarer Gruppen modifiziert. Dies erscheint umso überraschendér als nicht anzunehmen war, daß Beschichtungseigenschaften, die üblicherweise durch Wahl der Harzkomponenten festgelegt werden, durch den verwendeten Härtungskatalysator überhaupt in nennenswertem Maß beeinflußt werden können.

Gegenstand der Erfindung ist die Verwendung von Verbindungen der Formel

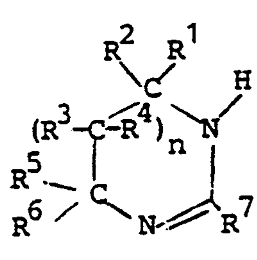

(I)

worin

n Null oder 1,

$R^1$-$R^6$ unabhängig voneinander ein Wasserstoffatom, $C_1$-$C_4$-Alkyl, $C_5$-$C_{10}$-Cycloalkyl, $C_7$-$C_{13}$-Aralkyl und $C_6$-$C_{18}$-Aryl oder zwei Substituenten $R^1/R^2$, $R^3/R^4$, $R^5/R^6$, $R^1/R^3$ oder $R^1/R^5$ zusammen einen $C_1$-$C_5$-Alkylenrest,

$R^7$ einen aliphatischen, cycloaliphatischen, araliphatischen oder aromatischen Rest mit 1 bis 36 C-Atomen, der entweder durch OH, COOH, $OR^8$, $NR_9R_{10}$ substituiert oder durch eine Ketogruppe oder 1 bis 10 Amid- oder Estergruppen unterbrochen ist oder einen polyesterrest mit der Struktur

$$-\underset{R^{11}}{CH}-(OCH_2-\underset{R^{11}}{CH})_{p-1}OCH_2CH_2R^{11},$$

$R^8$ $C_1$-$C_{15}$-Alkyl, $C_6$-$C_{19}$-Aryl,
$R^9$, $R^{10}$ unabhängig voneinander ein Wasserstoffatom, $C_1$-$C_{12}$-Alkyl, $C_6$-$C_8$-Cycloalkyl oder beide Substituenten zusammen $C_4$-$C_6$-Alkylen,
$R^{11}$ ein Wasserstoffatom, $C_1$-$C_4$-Alkyl, Phenyl und
p eine Zahl von 1 bis 40 bedeuten,
als Katalysatoren für die Härtung von, vorzugsweise pulverförmigen, Epoxidharzen.

Bevorzugte Verbindungen (1) sind substituierte Imidazoline und substituierte Tetrahydropyrimidine, insbesondere aber Amidine der Formel

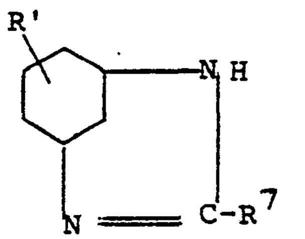

(1a)

worin
$R^7$ die oben angegebene Bedeutung hat und
R' einen $C_1$-$C_4$-Alkylrest, vorzugsweise Methyl, oder ein Wasserstoffatom bedeutet.

Weiterer Gegenstand der Erfindung sind Verbindungen der Formel

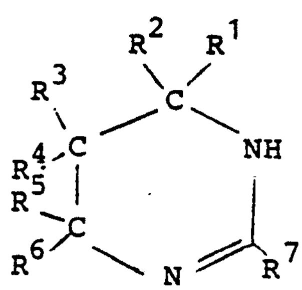

(2)

worin die Substituenten die oben angegebene Bedeutung haben.

Weiterer Gegenstand der Erfindung ist ein Verfahren zur Herstellung der Verbindungen (2), dadurch gekennzeichnet, daß man eine Carbonsäure der Formel R -COOH oder deren reaktionsfähiges Carbonsäurederivat mit 1,0 bis 2,5, vorzugsweise 1,0 bis 1,5 Mol, bezogen auf 1 Mol eingesetzte(s) Carbonsäure(derivat), Diamin der Formel
$H_2N$-$C(R^1R^2)$-$C(R^3R^4)$-$C(R^5R^6)$-$NH_2$
bei 150 bis 280, vorzugsweise 200 bis 250, °C umsetzt.

Bevorzugte Carbonsäuren $R^7$-COOH für die Herstellung der Verbindungen (1) sind z.B. Hydroxycarbonsäuren, wie z.B. Milchsäure, Ricinolsäure, 12-Hydroxystearinsäure, 9,10-Di-hydroxystearinsäure und Hydroxybenzoesäuren; Dicarbonsäuren, wie z.B. Adipinsäure, Azelainsäure,Iso- und Terephthalsäure; Aminosäuren, wie z.B. Glycin, Alanin, Sarkosin, Valin, Aminocapronsäure, Leucin und Isoleucin; Ethercarbonsäuren und Polyethercarbonsäuren; Ketocarbonsäuren, wie z.B. Lävulinsäure und Benzophenoncarbonsäuren; Amidgruppen-haltige Carbonsäuren der Formel

$$HO_2C-R^{12}-\underset{\underset{O}{\|}}{C}-\left[NH-R^{13}-NH-CO-R^{14}-\underset{\underset{O}{\|}}{C}-\right]_q-NH-R^{15} \qquad (3)$$

worin

q Null oder eine ganze Zahl von 1 bis 10, vorzugsweise, Null oder eine ganze Zahl von 1 bis 4,

$R^{12}$ -$R^{14}$ unabhängig voneinander zweiwertige aliphaische $C_2$-$C_{20}$-, vorzugsweise $C_4$-$C_{10}$-, oder aromatische $C_6$-$C_{19}$-, vorzugsweise $C_6$-$C_{15}$-Kohlenwasserstoffreste, wobei $R^1$ vorzugsweise für $C_6$-Alkyl, $C_6$-$C_{15}$-Cycloalkyl oder $C_6$-$C_{13}$-Aryl steht,

$R^{15}$ einen einwertigen $C_1$-$C_{18}$-Alkylrest oder einen $C_6$-$C_{15}$-Arylrest bedeuten;

Estergruppenhältige Carbonsäuren der Formel

$$HO_2C-R^{12}-\underset{\underset{O}{\|}}{C}-\left[OR^{13}O\overset{\overset{O}{\|}}{C}R^{14}\overset{\overset{O}{\|}}{C}-\right]_q OR^{15} \qquad (4)$$

worin

q und die Reste $R^{12}$-$R^{15}$ die oben angegebene Bedeutung haben, wobei aber vorzugsweise

$R^{12}$, $R^{14}$ unabhängig voneinander einen zweiwertigen aliphatischen $C_2$-$C_{10}$-Rest oder einen $C_6$-Arylrest,

$R^{13}$ einen zweiwertigen aliphatischen $C_2$-$C_6$-Rest

und

$R^{14}$ einen zweiwertigen aliphatischen $C_1$-$C_{18}$-Rest bedeuten.

Bei zweikernigen aromatischen Dicarbonsäuren können die Kerne auch durch ein Sauerstoffatom, eine Ketogruppe, eine Sulfogruppe oder eine $C_1$-$C_4$-Alkylengruppe verbunden sein.

Die oben genannten Polyethercarbonsäuren entsprechen vorzugsweise der Formel

$$R^{11}CH_2CH_2O-\left(\underset{R^{11}}{\underset{|}{CH}}-CH_2O\right)_{p-1}-\underset{R^{11}}{\underset{|}{CH}}-COOH$$

und können durch Cyanethylierung von Polyetherdiolmonoalkylethern und nachfolgende Hydrolyse des Nitrils sowie durch Oxidation der Hydroxylgruppe solcher polyetherdiolmonoalkylether (z.B. mit Permanganat, Chromsäureanhydrid, Wasserstoffperoxid und Katalysatoren, peroxiden usw.) hergestellt werden.

Bevorzugte Diamine für die Herstellung der Verbindungen (1) sind aliphatische und cycloaliphatische 1,2- und 1,3-Diamine, insbesondere Ethylendiamin, 1,2-Propylen-diamin, 1,3-Propylendiamin, 1,3-Diaminocyclohexane undbesonders bevorzugt- die verschiedenen Methyl-1,3-di-aminocyclohexan-Isomeren, die durch Hydrierung der technisch leicht zugänglichen Toluylendiamin-Isomerengemische erhalten werden können.

Ein Überschuß an Diamin hat sich als vorteilhaft erwiesen, da er die Bildung von Nebenprodukten, insbesondere von Diamiden, weniger begünstigt. In manchen Fällen ist es empfehlenswert, einen Katalysator mitzuverwenden.

Die Umsetzung kann abgebrochen werden, wenn keine eingesetzte Carbonsäure bzw. kein eingesetztes Carbonsäurederivat mehr nachweisbar ist und überschüssiges Diamin abdestilliert ist. In der Regel ist dies nach 2 bis 10 Stunden der Fall.

In vielen Fällen können die erhaltenen Amidine (1) ohne weitere Reinigung verwendet werden, da die

entstandenen Nebenprodukte (Amidamine, Diamide) die katalytische Aktivität der Amidin-Komponenten nicht nennenswert beeinflussen.

Zur Erhöhung des Schmelzpunktes der Verbindungen (1) kann - soweit möglich - eine reversible Umsetzung mit Poly- oder vorzugsweise Monoisocyanaten hilfreich sein; die Vernetzung der Pulverlacke wird auf diese Weise zu höheren Temperaturen hin verschoben.

Die erfindungsgemäßen Katalysatoren werden in Mengen von 0,1 bis 5, vorzugsweise von 1 bis 2, Gew.-%, bezogen auf die Summe von Epoxidharz und dem gegebenenfalls vorhandenen sauren Polymeren, eingesetzt. Die Zugabe wird vorzugsweise in einem Arbeitsgang bei der Herstellung des gebrauchsfertigen Pulverlacksystems durch Extrusion vorgenommen.

Die verwendbaren polyepoxide sind feste, zumeist harzartige Stoffe, die im Bereich von 30 bis 140°C, vorzugsweise zwischen 40 und 80°C (bestimmt nach der Methode der Differentialthermoanalyse) schmelzen und die im Durchschnitt mehr als eine 1,2-Epoxygruppe pro Molekül enthalten.

Zum einen handelt es sich hierbei um Polyepoxidverbindungen auf Basis mehrwertiger Phenole, beispielsweise aus Brenzkatechin, Resorcin, Hydrochinon, aus 4,4'-Di-hydroxydiphenylmethan, aus 4,4'-Dihydroxy-3,3'-dime-thyl-diphenylmethan, aus 4,4'-Dihydroxydiphenyldimethyl-methan (Bisphenol A), aus 4,4'-Dihydroxydiphenylmethylmethan, aus 4,4'-Dihydroxydiphenylcyclohexan, aus 4,4'-Dihydroxy-3,3'-dimethyldiphenylpropan, aus 4,4'-Dihy-droxydiphenyl, aus 4,4'-Dihydroxydiphenylsulfon, aus Tris-(4-hydroxyphenyl)-methan, aus den Chlorierungsund Bromierungsprodukten der vorstehend genannten Diphenole, insbesondere aus Bisphenol A; aus Novolaken (d.h. aus Umsetzungsprodukten von ein- oder mehrwertigen Phenolen mit Aldehyden, insbesondere Formaldehyd, in Gegenwart saurer Katalysatoren), aus Diphenolen, die durch Veresterung von 2 Mol des Natriumsalzes einer aromatischen Oxycarbonsäure mit einem Mol eines Dihalogenalkans oder Dihalogendialkylethers erhalten wurden (vgl. britische Patentschrift 1 017 612), aus polyphenolen, die durch Kondensation von phenolen und langkettigen, mindestens 2 Halogenatome enthaltenden Halogenparaffinen erhalten wurden (vgl. britische Patentschrift 1 024 288).

Bevorzugt werden handelsübliche, feste Epoxidharze des Typs Diglycidylether des Bisphenols A (d.h. Umsetzungsprodukte von Bisphenol A mit Epichlorhydrin), deren Epoxidäquivalentgewicht zwischen 400 und 2500 liegt, eingesetzt.

Weiter kommen Verbindungen wie (Poly)glycidylester der Formel

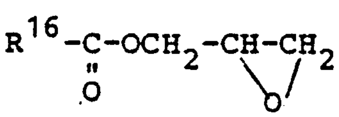 (5)

in Frage, in denen $R^{16}$ ein gradkettiger oder verzweigtkettiger, gesättigter oder ungesättigter Kohlenwasserstoffrest mit 4-20 Kohlenstoffatomen oder ein gegebenenfalls substituierter Phenylrest ist.

Ferner können als Epoxidharz Verbindungen wie Triglycidylisocyanurat und/oder dessen Oligomere und Triglycidylurazol sowie dessen Oligomere sowie Mischungen der genannten Stoffklassen eingesetzt werden.

Die carboxylgruppenhaltigen Polymeren können Polyesterpolycarbonsäuren sein, welche aus Polyolen und Polycarbonsäuren bzw. deren Derivaten hergestellt werden.

Die carboxylgruppenhaltigen polymeren sollen einen Schmelz- und Erweichungsbereich (bestimmt durch Differentialthermoanalyse) von 20°C - 150°C, vorzugsweise 50°C - 100°C, und eine Säurezahl von 10 - 150, vorzugsweise 20 - 120, insbesondere von 30 - 50, haben. Die OH-Zahlen sollen vorzugsweise unter 20, insbesondere unter 10, liegen.

Die Veresterungsreaktion zum Aufbau der Polyestercarbonsäuren kann nach bekannten Verfahren durch Veresterung entsprechender Polycarbonsäuren und Polyole, insbesondere von Dicarbonsäuren und Dialkoholen oder durch Esterbildung aus geeigneten Derivaten dieser Alkohole und Carbonsäuren, wie z.B. der Anhydride, Säurechloride sowie auch mit Hydroxycarbonsäuren durchgeführt werden.

Durch den Einbau von mindestens trifunktionellen Polycarbonsäuren bzw. deren Anhydriden, wie Benzol-1,3,5-tricarbonsäure oder Trimellithsäureanhydrid werden besonders bevorzugte verzweigte, d.h. mindestens trisfunktionelle Polyester-polycarbonsäuren erhalten.

Das molare Mischungsverhältnis von carboxylgruppenhaltigen polymeren zu Epoxidharz wird in der Regel so gewählt, daß auf eine freie Carboxylgruppe 0,6 bis 1,5, vorzugsweise 0,8 bis 1,25, Epoxidgruppen kommen.

Pulverlackhilfsmittel und -zusätze, wie z.B. Pigmente, Farbstoffe, Füllstoffe, Verlaufsmittel, Thixotropiermittel, Entlüftungsmittel, UV-Stabilisatoren und Oxidationsinhibitoren und Quencher (Radikal-Fänger, wie z.B. N-alkylsubstituierte Piperidine) sowie Mattierungsmittel und solche Mittel, die die Oberflächenglätte verbessern, können selbstverständlich, wie üblich, eingearbeitet werden.

Die Herstellung von Pulverlacken geschieht üblicherweise wie folgt:

Das carboxylgruppenhaltige Polymere wird mit dem gewählten Polyepoxid und gegebenenfalls weiteren Zusätzen zunächst gemischt und in der Schmelze homogenisiert. Dies kann in geeigneten Aggregaten, wie z.B. beheizbaren Knetern, vorzugsweise jedoch durch Extrudieren, erfolgen, wobei die Extrusionstemperatur so zu wählen ist, daß ein Maximum an Scherkraft auf die Mischung einwirkt. Dabei sollte eine Temperaturobergrenze von 140°C nicht überschritten werden.

Die extrudierte Masse wird nach Abkühlung auf Raumtemperatur und nach einer geeigneten Vorzerkleinerung zu einem Pulverlack gemahlen, wobei je nach dem Verwendungszweck mittlere Teilchengrößen von ca. 40-90 µm, vorzugsweise jedoch ca. 50 µm, angestrebt werden. Ein eventuell vorhandener Grobkornanteil von Teilchengrößen von mehr als 90 µm wird durch Absieben entfernt.

Das Auftragen der so hergestellten Pulverlacke auf geeignete Substrate kann nach den bekannten Verfahren, wie z.B. durch elektrostatisches Pulversprühen, Wirbelsintern, elektrostatisches Wirbelsintern sowie im Flammspritzverfahren oder durch das Auftragen von wäßriger Suspension nach konventionellen oder elektrischen Verfahren erfolgen.

Nach dem Auftragen des Pulverlackes nach einem der genannten Verfahren werden die beschichteten Werkstücke zur Aushärtung auf Temperaturen von 140°C oder höher erhitzt, wobei sich die Zeitdauer der Erhitzung im wesentlichen nach der Wärmekapazität des beschichteten Werkstückes oder dessen Temperatur vor der Beschichtung richtet.

Viele der polar modifizierten Amidine sind in Lösungsmitteln, wie z.B. Benzylalkohol, sehr gut löslich - weit besser als die nicht polar modifizierten Analoga - und können in dieser Form auch sehr gut in flüssigen Epoxysystemen als Härtungskatalysatoren verwendet werden. Die COOH- und OH-modifizierten Amidine (1) lassen sich gut in Wasser dispergieren und können somit auch in umweltfreundlichen wäßrigen Beschichtungssystemen eingesetzt werden.

In den nachfolgenden Beispielen angegebene Teile und Verhältnisse beziehen sich, falls nicht anders angegeben, auf das Gewicht.

**Beispiel 1**

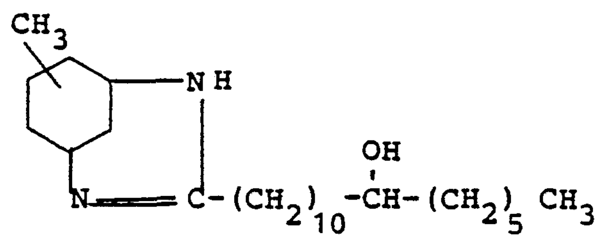

(6)

In einem 250 ml Dreihalskolben, versehen mit Rührer, Thermometer und Claisenbrücke mit einem 100 ml Vorlagekolben, wurden 64 g (0,5 Mol) Methyl-2,4-diamino-cyclo-hexan (Isomerengemisch), im folgenden "PH-Tolamin" genannt, und 80 g (0,25 Mol) 12-Hydroxystearinsäure unter Stickstoffatmosphäre vorgelegt. Sodann wurde unter Rühren zügig auf 250°C aufgeheizt, wobei Wasser und überschüssiges PH-Tolamin abdestillierten. Nach einer Reaktionszeit von 4 Stunden bei 250°C war die Reaktion beendet.

Man erhielt neben 39,6 g Destillat (überschüssiges PH-Tolamin und Wasser) 104 g eines bräunlichen Wachses mit einem Erweichungsbereich um 40°C, das ohne weitere Reinigung als Härtungskatalysator verwendet werden konnte.

**Beispiel 2**

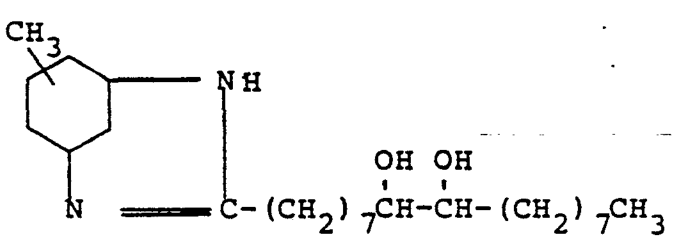

(7)

Analog Beispiel 1 wurden 77 g (0,6 Mol) PH-Tolamin und 116 g (0,35 Mol) 9,10-Dihydroxystearinsäure bei 250°C innerhalb von 4 Stunden miteinander umgesetzt. Neben 61 g Destillat erhielt man 131,5 g eines gelben Harzes.

**Beispiel 3**

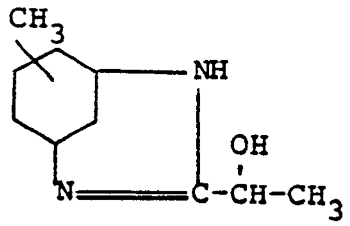

(8)

In einem 500 ml Dreihalskolben, versehen mit Rührer, Thermometer, Tropftrichter und Claisenbrücke mit einem 250 ml Vorlagekolben wurden 190 g (1,5 Mol) PH-Tolamin unter Rühren und Stickstoffatmosphäre vorgelegt. 100 g (1 Mol) wäßrige Milchsäure (90 %ig) wurden innerhalb von 15 Minuten portionsweise eingetragen. Die Temperatur stieg dabei auf 105°C an. Anschließend wurde zügig auf 250°C aufgeheizt, wobei Wasser und überschüssiges PH-Tolamin abdestillierten. Nach 4 Stunden Reaktionszeit bei 250°C war die Reaktion beendet. Man erhielt 143 g Destillat (PH-Tolamin und Wasser) und 145 g eines rot-braun kristallinen Reaktionsproduktes mit einem Fp. von 50°C. Das Reaktionsprodukt konnte ohne weitere Reinigung als Härtungskatalysator verwendet werden.

**Beispiel 4**

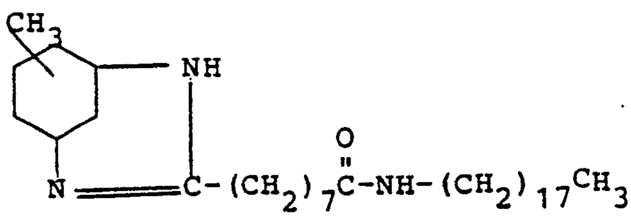

(9)

Bei 80°C wurden zu 439 g (1,0 Mol) Azelainsäurestearylamid 190 g (1,5 Mol) PH-Tolamin zugesetzt und die Mischung unter Stickstoffatmosphäre und Rühren auf 250°C aufgeheizt, wobei Wasser und überschüssiges PH-Tolamin abdestillierten. Nach 4 Stunden bei 250°C war die Reaktion beendet. Man erhielt 63 g Destillat und 565 g eines hellgelben, spröden Harzes als Reaktionsprodukt (Erweichungspunkt ca. 80°C). Das Reaktionsprodukt konnte ohne weitere Reinigung als Härtungskatalysator verwendet werden.

**Beispiel 5**

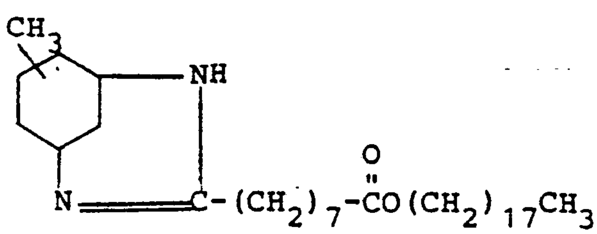

(10)

Bei 80°C wurden zu 438 g (1,0 Mol) Azelainsäuremonostearylester 190 g (1,5 Mol) PH-Tolamin zugesetzt und die Mischung unter Stickstoffatmosphäre und Rühren auf 250°C aufgeheizt, wobei Wasser und überschüssiges PH-Tolamin abdestillierten. Nach 4 Stunden Nachrühren bei 250°C war die Reaktion beendet. Man erhielt 47 g Destillat und 580 g eines hellen Wachses mit einem Erweichungspunkt von ca. 40°C.

**Beispiel 6**

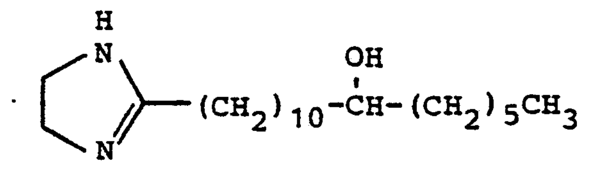

(11)

Analog Beispiel 1 wurden 60 g (1 Mol) Ethylendiamin und 126 g (0,4 Mol) 12-Hydroxystearinsäure unter Stickstoffatmosphäre und Rühren auf 250°C erhitzt, wobei Wasser und überschüssiges Ethylendiamin abdestillierten. Nach 4 Stunden Reaktionszeit bei 250°C war die Reaktion beendet.

Neben 50 g Destillat erhielt man 135 g hellgelbe Kristalle mit einem Erweichungsbereich um ca. 110°C. Das Reaktionsprodukt konnte ohne weitere Reinigung als Härtungskatalysator verwendet werden.

**Verwendungsbeispiele, Herstellung von Pulverlacken**

**Beispiel 7**

60,9 Teile eines Carboxylpolyesters aus Neopentylglykol, Terephthalsäure, Isophthalsäure, Trimellitsäureanhydrid, mit einem Erweichungspunkt von 75°C (DTA) und einer Säurezahl von 35 wurden mit 0,62 Teilen des Amidins nach Beispiel 1 und mit 4,6 Teilen Triglycidylisocyanurat (entsprechend einem Verhältnis polyesterharz/Epoxidhärter = 93: 7) sowie mit 33,0 Teilen eines hochbeständigen Titandioxid-Rutils und mit 0,4 Teilen eines Verlaufmittels auf Basis eines handelsüblichen Acrylatoligomeren zunächst trocken gemischt. Diese Mischung wurde auf einem Laborextruder bei Temperaturen von 80-120°C in der Schmelze dispergiert. Das Extrudat wurde nach Abkühlung und Vorzerkleinerung auf einer Gebläsemühle auf eine durchschnittliche Korngröße von 50 µm zu einem Pulverlack gemahlen. Nach Absieben des vorhandenen Grobkornanteils von Teilchengrößen oberhalb 90 µm wurde der gebrauchsfertige Pulverlack auf doppelt dekapierte, entfettete Prüfbleche (Länge 16,5 cm, Breite 65 mm, Stärke 0,8 mm) mit einer elektrostatischen Sprühvorrichtung bei einer Negativspannung von ca. 60 kV aufgesprüht. Die Bleche wurden anschließend bei verschiedenen Temperaturen eingebrannt.

Die Beschichtungen wurden in einer Stärke von 50 µm nach den folgenden praxisüblichen Methoden auf ihre Eigenschaften geprüft:

Erichsentiefung (DIN 53 156) → "Tiefung"
Glanz, 60° (DIN 67 530) → "Glanz"
Impact reverse (Kugel Ø 12,7 mm) → IMR, 1 kg
Acetonfestigkeit, beurteilt nach folgender Abstufung:
- 1..... -50 Anzahl Hübe mit Aceton getränktem Wattebausch bis zum Durchscheuern des Lackfilms
2 m Tach 50 Hüben gatter, weicher Film
2 lm nach 50 Hüben leicht matter, weicher Film
2 weicher Film nach 50 Hüben,
1 leicht oberflächenempfindlicher Lackfilm nach 50 Hüben
0 keine Veränderungen
Verlauf: Visuelle Beurteilung der Lackoberfläche.

Die Gitterschnittprüfung nach DIN 53 151 ergab in allen Beispielen und Vergleichsversuchen die gute Beurteilung Gt 0/0.

Die prüfergebnisse sind in Tabelle 1 zusammengestellt.

**Beispiel 8**

Analog Beispiel 7 wurde ein Pulverlack mit 0,62 Teilen des Amidins aus Beispiel 6 als Katalysator hergestellt.

**Vergleichsversuche 1 und 2**

Analog Beispiel 7 wurden Pulverlacke hergestellt, wobei jedoch als Katalysatoren 0,62 Teile eines Amidins (12) (VV 1) bzw. 0,62 Teile 2-Phenylimidazolin (VV 2) verwendet wurden.

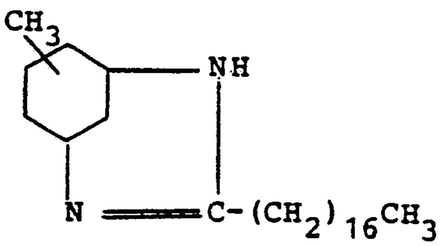

(12)

**Beispiel 9**

Aus 39,0 Teilen des Carboxylpolyesters aus Beispiel 7, 26,0 Teilen eines Epoxydharzes mit einem Epoxydäquivalent von 800 und einem Schmelzbereich von 90-100°C (Verhältnis Polyester/Epoxyd 60:40) und 0,2 Teilen eines Amid-Amidins nach Beispiel 4 sowie 33,0 Teilen eines hochbeständigen Titandioxyd-Rutils und 0,6 Teilen eines Verlaufmittels auf Basis eines handelsüblichen Acrylatoligomeren wurde ein Pulverlack hergestellt.

**Beispiel 10**

Analog Beispiel 9 wurde ein Pulverlack hergestellt mit einem Ester-Amidin nach Beispiel 5.

Tabelle 1

| | | Beispiel 7 | VV1 | Beispiel 8 | VV2 | Beispiel 9 | Beispiel 10 |
|---|---|---|---|---|---|---|---|
| Gelierzeit bei 180°C (sec) | | 68 | 91 | 76 | 348 | 164 | 150 |
| Tiefung | 30 min/140°C | >10 | >10 | >10 | 0,3 | 8,0 | >10 |
| (mm) | 10 min/160°C | >10 | >10 | >10 | 0,3 | | |
| | 20 min/160°C | | | | | >10 | >10 |
| | 15 min/170°C | | | | | >10 | >10 |
| IMR | 30 min/140°C | 150 | 80 | -5 | -5 | 10 | 5 |
| (cm kg) | 10 min/160°C | 120 | 10 | -5 | -5 | | |
| | 20 min/160°C | | | | | 150 | 150 |
| | 15 min/170°C | | | | | 140 | 140 |
| Glanz | 30 min/140°C | 90 | 85 | 91 | 92 | 99 | 99 |
| 60° * | 10 min/160°C | 91 | 91 | 91 | 91 | | |
| | 20 min/160°C | | | | | 99 | 98 |
| | 15 min/170°C | | | | | 98 | 98 |
| Aceton- | 30 min/140°C | 0-1 | 2 | 0-1 | -6 | -40 | 2 m |
| festig- | 10 min/160°C | 1 | 1 | 2 | -6 | | |
| keit | 20 min/160°C | | | | | 2 m | 2 m |
| | 15 min/170°C | | | | | 2 lm | 2 |
| Verlauf (visuell) | | ← leichte Struktur → | | | | | |

**Beispiel 11**

Analog Beispiel 7 wurde ein Pulverlack hergestellt aus 62,4 Teilen eines Epoxydharzes mit einem Epoxydäquivalent von 800 und einem Schmelzbereich von 90-100°C mit 4,0 Teilen des Amidins aus Beispiel 3 sowie mit 33 Teilen eines hochbeständigen Titandioxid-Rutils und mit 0,6 Teilen eines Verlaufmittels auf Basis eines Acrylatoligomeren.

**Vergleichsversuche 3 und 4**

Analog Beispiel 11 wurde ein Pulverlack hergestellt, wobei aber 4,0 Teile eines Amidins nach Formel (12) (VV3) bzw. 0,62 Teile 2-Phenylimidazolin (VV4) verwendet wurden.

**Beispiel 12**

Analog Beispiel 11 wurde mit 0,62 Teilen Amidin nach Beispiel 2 ein Pulverlack hergestellt.

**Beispiel 13**

Analog Beispiel 11 wurde ein Pulverlack aus 62,4 Teilen Epoxidharz mit einem Äquivalent von 800 und 4,0 Teilen des Amidins aus Beispiel 3 mit 0,2 Teilen eines Emulgators auf Basis oxyethyliertem Nonylphenol, mit 33,0 Teilen eines hochbeständigen Titandioxid-Rutil-Typs und 6,0 Teilen eines Polypropylenglykols

(Molekulargewicht ca. 1000) und 0,6 Teilen eines Acrylatoligomeren hergestellt.

Dieser Pulverlack wurde in Wasser dispergiert und in einer Kugelmühle auf eine Feinheit von ca. 5 µm gemahlen. Diese Suspension wurde mit einer normalen Luftpistole auf Aluminium aufgetragen.

**Vergleichsversuch 5**

Analog Beispiel 13 wurde eine Pulverlacksuspension mit 4,0 Teilen eines Amidins der Formel (12) hergestellt.

Tabelle 2

| | | Beispiel 11 | VV3 | VV4 | Beispiel 12 | Beispiel 13 | VV5 |
|---|---|---|---|---|---|---|---|
| Gelierzeit bei 180°C (sec) | | 50 | 75 | 35 | 35 | 50 | 75 |
| Tiefung | 30 min/140°C | > 10 | > 10 | 6,0 | 8,7 | 7,3 | 6,0 |
| (mm) | 20 min/150°C | >10 | 8,8 | 6,0 | 8,8 | 6,9 | 7,3 |
| | 8 min/170°C | >10 | 9,8 | 5,8 | 9,1 | 9,6 | 8,0 |
| IMR | 30 min/140°C | 130 | 5 | -5 | 150 | 5 | 5 |
| (cm kg) | 20 min/150°C | 130 | 50 | 20 | 80 | 5 | -5 |
| | 8 min/170°C | 80 | -5 | 60 | 60 | 5 | 5 |
| Glanz | 30 min/140°C | 102 | 95 | 95 | 94 | 90 | 90 |
| 60° ∢ | 20 min/150°C | 101 | 95 | 95 | 91 | 90 | 89 |
| | 8 min/170°C | 101 | 91 | 92 | 89 | 86 | 86 |
| Aceton- | 30 min/140°C | 0-1 | 0-1 | 0-1 | 0-1 | 1 | 1 |
| festig- | 20 min/150°C | 0-1 | 0-1 | 0-1 | 0-1 | 1 | 1 |
| keit | 8 min/170°C | 0-1 | 1 | 0-1 | 0-1 | 0-1 | 1 |
| Verlauf (visuell) | | leichte Struktur | Struktur | starke Struktur | leichte Struktur | kraterfreie Oberfläche | viele Krater und Nadelstriche |

**Patentansprüche**

1. Verwendung von Verbindungen der Formel

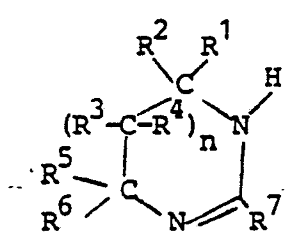

(1)

n Null oder 1,

$R^1$-$R^6$ unabhängig voneinander ein Wasserstoffatom, $C_1$-$C_4$-Alkyl, $C_5$-$C_{10}$-Cycloalkyl, $C_7$-$C_{13}$-Aralkyl und $C_6$-$C_{18}$-Aryl oder zwei Substituenten $R^1/R^2$, $R^3/R^4$, $R^5/R^6$, $R^1/R^3$ oder $R^1/R^5$ zusammen einen $C_1$-$C_5$-Alkylenrest,

$R^7$ einen aliphatischen, cyclialiphatischen, araliphatischen oder aromatischen Rest mit 1 bis 36 C-Atomen, der entweder durch OH, COOH, $OR^8$, $NR^9R^{10}$ substituiert oder durch eine Ketogruppe oder 1 bis 10 Amidoder Estergruppen unterbrochen ist, oder einen Polyetherrest mit der Struktur

$$-\underset{R^{11}}{\underset{|}{CH}}-(OCH_2-\underset{R^{11}}{\underset{|}{CH}})_{p-1}OCH_2CH_2R^{11}$$

$R^8$ $C_1$-$C_{15}$-Alkyl, $C_6$-$C_{19}$-Aryl,

$R^9$,$R^{10}$ unabhängig voneinander ein Wasserstoffatom, $C_1$-$C_{12}$-Alkyl, $C_6$-$C_8$-Cycloalkyl oder beide Substituenten zusammen $C_4$-$C_6$-Alkylen,

$R^{11}$ ein Wasserstoffatom, $C_1$-$C_4$-Alkyl, Phenyl und

p eine Zahl von 1 bis 40 bedeuten,

als Katalysatoren für die Härtung von vorzugsweise pulverförmigen Epoxidharzen.

2. Verbindungen der Formel

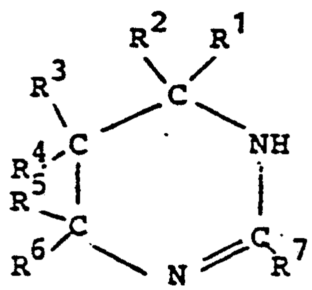

(2)

worin

$R^1$-$R^6$ unabhängig voneinander ein Wasserstoffatom, $C_1$-$C_4$-Alkyl, $C_5$-$C_{10}$-Cycloalkyl, $C_7$-$C_{13}$-Aralkyl und $C_6$-$C_{18}$-Aryl oder zwei Substituenten $R^1/R^2$, $R^3/R^4$, $R^5/R^6$, $R^1(R^3$ oder $R^1/R^5$ zusammen einen $C_1$-$C_5$-Alkylen-rest,

$R^7$ einen aliphatischen, cycloaliphatischen oder aromatischen Rest mit 1 bis 36 C-Atomen, der entweder durch COOH substituiert ist oder einen Polyetherrest mit der Struktur

$$-\underset{R^{11}}{\underset{|}{CH}}-(OCH_2-\underset{R^{11}}{\underset{|}{CH}})_{p-1}OCH_2CH_2R^{11}$$

einen Amidgruppen-haltigen Rest der Formel

$$-R^{12}-\underset{O}{\underset{\|}{C}}-\left[NH-R^{13}-NH-CO-R^{14}-\underset{O}{\underset{\|}{C}}-\right]_q-NH-R^{15}$$

oder einen Estergruppen-haltigen Rest der Formel

$$-R^{12}-\underset{O}{\underset{\|}{C}}-\left[OR^{13}O\overset{O}{\overset{\|}{C}}R^{14}\overset{O}{\overset{\|}{C}}-\right]_q OR^{15}$$

oder einen der Reste der Formeln

$$CH_3 \cdot [CH_2]_5 \cdot \underset{OH}{CH} \cdot CH_2 \cdot CH = CH \cdot [CH_2]_7-,$$

$$CH_3 \cdot [CH_2]_5 \cdot \underset{OH}{CH} \cdot [CH_2]_{10}-,$$

$$CH_3 \cdot [CH_2]_7 \cdot \underset{OH}{CH} \cdot \underset{OH}{CH} \cdot [CH_2]_7-,$$

$R^{11}$ einen Wasserstoff, $C_1$ - $C_4$-Alkyl, Phenyl,
p eine Zahl von 1 bis 40,
q Null oder eine ganze Zahl von 1 bis 10,
$R^{12}$ - $R^{14}$ unabhängig voneinander zweiwertige aliphatische $C_2$-$C_{20}$ oder aromatische $C_6$-$C_{19}$-Kohlenwasserstoffreste, wobei R vorzugsweise für $C_6$-Alkyl, $C_6$-$C_{15}$-Cycloalkyl oder $C_6$-$C_{13}$-Aryl steht,
$R^{15}$ einen einwertigen $C_1$-$C_{18}$-Alkylrest oder einen $C_6$-$C_{15}$-Arylrest bedeuten.

3. Verbindungen der Formel

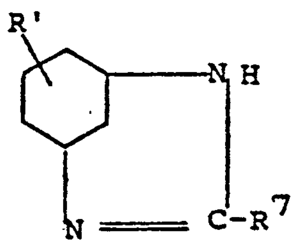

(1a)

worin R' einen $C_1$-$C_4$-Alkylrest oder ein Wasserstoffatom und
$R^7$ die in Anspruch 2 angegebene Bedeutung hat.

4. Verbindungen nach Ansprüchen 2 und 3, dadurch gekennzeichnet, daß R' für eine Methylgruppe steht.

5. Verfahren zur Herstellung der Verbindungen nach Ansprüchen 2-4, dadurch gekennzeichnet, daß man eine Carbonsäure der Formel $R^7$-COOH oder deren reaktionsfähiges Carbonsäurederivat mit 1,0 bis 2,5 Mol, bezogen auf 1 Mol eingesetzte(s) Carbonsäure(derivat), Diamin der Formel
$H_2N$-$C(R^1R^2)$-$C(R^3R^4)$-$C(R^5R^6)$-$NH_2$
bei 150 bis 280°C umsetzt.

## Claims

1. Use of compounds of the formula

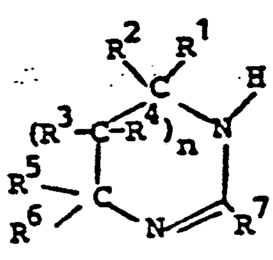

(1)

wherein

n denotes zero or 1,

$R^1$-$R^6$ independently of one another denote a hydrogen atom, $C_1$-$C_4$-alkyl, $C_5$-$C_{10}$-cycloalkyl, $C_7$-$C_{13}$-aralkyl and $C_6$-$C_{18}$-aryl or two substituents $R^1/R^2$, $R^3/R^4$, $R^5/R^6$, $R^1/R^3$ or $R^1/R^5$ together denote a $C_1$-$C_5$-alkylene radical,

$R^7$ denotes an aliphatic, cycloaliphatic, araliphatic or aromatic radical with 1 to 36 C atoms, which is either substituted by OH, COOH, $OR^8$ or $NR^9R^{10}$ or is interrupted by a keto group or 1 to 10 amide or ester groups, or a polyether radical with the structure

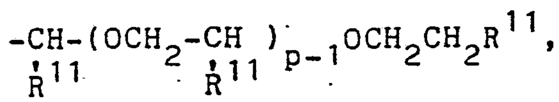

$R^8$ denotes $C_1$-$C_{15}$-alkyl or $C_6$-$C_{19}$-aryl,

$R^9$ and $R^{10}$ independently of one another denote a hydrogen atom, $C_1$-$C_{12}$-alkyl or $C_6$-C.-cycloalkyl or both substituants together denote $C_4$-$C_6$-alkylene,

$R^{11}$ denotes a hydrogen atom, $C_1$-$C_4$-alkyl or phenyl and

p denotes a number from 1 to 40,

as catalysts for hardening prefarably pulverulant epoxy resins.

2. Compounds of the formula

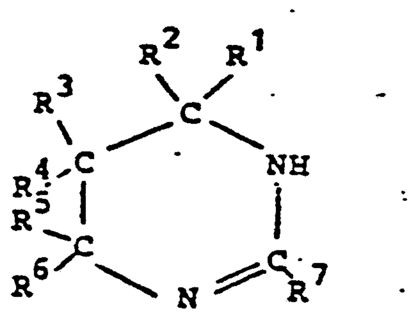

(2)

wherein

$R^1$-$R^6$ independently of one another denote a hydrogen atom, $C_1$-$C_4$-alkyl, $C_5$-$C_{10}$-cycloalkyl, $C_7$-$C_{13}$-aralkyl and $C_6$-$C_{15}$-aryl or two substituents $R^1/R^2$, $R^3/R^4$, $R^5/R^6$, $R^1/R^3$ or $R^1/R^5$ together denote a $C_1$-$C_5$-alkylene radical,

$R^7$ denotes an aliphatic, cycloaliphatic or aromatic radical with 1 to 36 C atoms which is either substituted by COOH, or a polyether radical with the structure

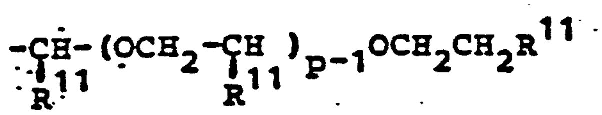

an amide-group-containing radical of the formula

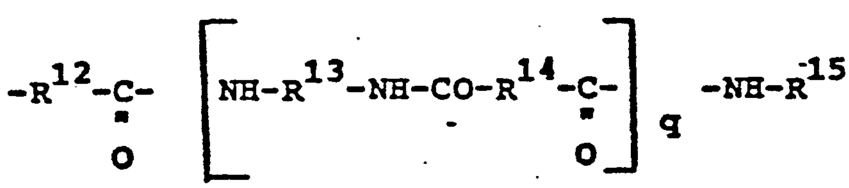

or an ester-group-containing radical of the formula

$$-R^{12}-\underset{\underset{O}{\|}}{C}-\left[OR^{13}O\overset{\overset{O}{\|}}{C}R^{14}\overset{\overset{O}{\|}}{C}-\right]_q OR^{15}$$

or one of the radicals of the formulae

$$CH_3\cdot[CH_2]_5\cdot\underset{\underset{OH}{|}}{CH}\cdot CH_2\cdot CH=CH\cdot[CH_2]_7-$$

$$CH_3\cdot[CH_2]_5\cdot\underset{\underset{OH}{|}}{CH}\cdot[CH_2]_{10}-\ ,$$

$$CH_3\cdot[CH_2]_7\cdot\underset{\underset{OH}{|}}{CH}\cdot\underset{\underset{OH}{|}}{CH}\cdot[CH_2]_7-\ ,$$

$R^{11}$ denotes a hydrogen, $C_1$-$C_4$-alkyl or phenyl,
p denotes a number from 1 to 40,
q denotes zero or an integer from 1 to 10,
$R^{12}$ - $R^{14}$ independently of one another denote /divalent aliphatic $C_2$-$C_{20}$ or aromatic $C_6$-$C_{19}$ hydrocarbon radicals, $R^{13}$ preferably representing $C_6$-alkyl, $C_6$-$C_{15}$-cycloalkyl or $C_6$-$C_{13}$-aryl, and
$R^{15}$ denotes a monovalent $C_1$-$C_{18}$-alkyl radical or a $C_6$-$C_{15}$-aryl radical.

3. Compounds of the formula

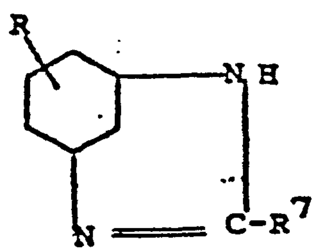

(1a)

wherein
R' a $C_1$-$C_4$-alkyl radical or à hydrogen atom and
$R^7$ has the meaning given in Claim 2.

4. Compounds according to Claims 2 and 3, characterised in that R' represents a methyl group.

5. Process for the preparation of the compounds according to Claims 2-4, characterised in that a carboxylic acid of the formula $R^7$-COOH or a reactive carboxylic acid derivative thereofis reacted with 1.0 to 2,5 mols basad on 1 mol of the carboxylic acid (derivative) used, of a diamine of the formula:
$H_2N$-$C(R^1R^2)$-$C(R^3R^4)$-$C(R^5R^6)$-$NH_2$
at 150 to 280° C.

**Revendications**

1. Utilisation de composé de formule:

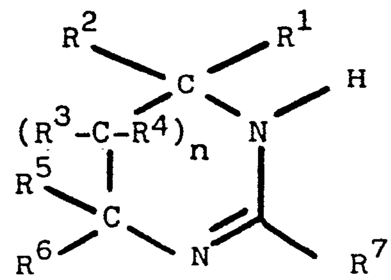

(1)

dans laquelle

n représente 0 ou 1,

$R^1$-$R^6$ représentent chacun indépendamment l'un de l'autre un atome d'hydrogène, un groupe alkyle en $C_1$-$C_4$, un groupe cycloalkyle en $C_5$-$C_{10}$, un groupe aralkyle en $C_7$-$C_{13}$ et un groupe aryle en $C_6$-$C_{18}$ ou deux substituants $R^1/R^2$, $R^3/R^4$, $R^5/R^6$, $R^1/R^3$ ou $R^1/R^5$ représentent ensemble un groupe alkylène en $C_1$-$C_5$,

$R^7$ représente un radical aliphatique, cycloaliphatique, araliphatique ou aromatique contenant 1 à 36 atomes de carbone, qui est substitué par OH, COOH, $OR^8$, $NR^9R^{10}$ ou qui est interrompu par un groupe céto ou 1 à 10 groupes amido ou ester, ou encore un radical polyéther ayant la structure

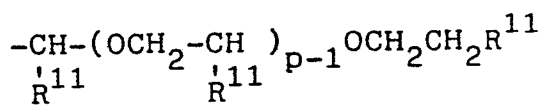

$R^8$ représente un groupe alkyle en $C_1$-$C_{15}$ ou un groupe aryle en $C_6$-$C_{19}$,

$R^9$, $R^{10}$ représentent chacun indépendamment l'un de l'autre un atome d'hydrogène, un groupe alkyle en $C_1$-$C_{12}$, un groupe cycloalkyle en $C_6$-$C_8$ ou les deux substituants ensemble représentent un groupe alkylène en $C_4$-$C_6$,

$R^{11}$ représente un atome d'hydrogène, un groupe alkyle en $C_1$-$C_4$ ou un groupe phényle et

p est un nombre de 1 à 40,

comme catalyseurs pour le durcissement de résines époxy, de préférence, pulvérulentes.

2. Composés de formule:

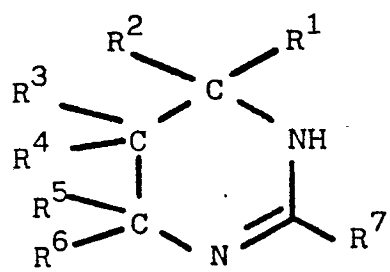

(2)

dans laquelle

$R^1$-$R^6$ représentent chacun indèpendamment l'un de l'autre un atome d'hydrogène, un groupe alkyle en $C_1$-$C_4$, un groupe cycloalkyle en $C_5$-$C_{10}$, un groupe aralkyle en $C_7$-$C_{13}$ et un groupe aryle en $C_6$-$C_{18}$ ou deux substituants $R^1/R^2$, $R^3/R^4$, $R^5/R^6$, $R^1/R^3$ ou $R^1/R^5$ ensemble représentent un groupe alkylène en $C_1$-$C_5$,

$R^7$ représente un radical aliphatique, cycloaliphatique ou aromatique contenant 1 à36 atomes de carbone qui est substitué par COOH ou un radical polyèther de structure

$$-\underset{R^{11}}{CH}-(OCH_2-\underset{R^{11}}{CH})_{p-1}OCH_2CH_2R^{11}$$

un radical contenant des groupes amido et répondant à la formule:

$$-R^{12}-\underset{O}{\overset{}{\underset{\|}{C}}}-\left[NH-R^{13}-NH-CO-R^{14}-\underset{O}{\underset{\|}{C}}-\right]_q-NH-R^{15}$$

ou un radical contenant des groupes ester et rèpondant à la formule:

$$-R^{12}-\underset{O}{\underset{\|}{C}}-\left[OR^{13}O\overset{O}{\overset{\|}{C}}R^{14}\overset{O}{\overset{\|}{C}}-\right]_q OR^{15}$$

ou un des radicaux rèpondant aux formules:

$$CH_3.[CH_2]_5.\underset{OH}{CH}.CH_2.CH{=}CH.[CH_2]_7- ,$$

$$CH_3.[CH_2]_5.\underset{OH}{CH}.[CH_2]_{10}- , \quad CH_3.[CH_2]_7.\underset{OH}{CH}.\underset{OH}{CH}.[CH_2]_7- ,$$

$R^{11}$ représente un atome d'hydrogène, un groupe alkyle en $C_1$-$C_4$ ou un groupe phényle,
p représente un nombre de 1 à 40,
q représente 0 ou un nombre entier de 1 à 10,
$R^{12}$-$R^{14}$ représentent chacun indépendamment l'un de l'autre des radicaux d'hydrocarbures aliphatiques bivalents en $C_2$-$C_{20}$ ou des radicaux d'hydrocarbures aromatiques en $C_6$-$C_{19}$, $R^{13}$ représentant, de préférence, un groupe alkyle en $C_6$, un groupe cycloalkyle en $C_6$-$C_{15}$ ou un groupe aryle en $C_6$-$C_{13}$,
$R^{15}$ représente un groupe alkyle monovalent en $C_1$-$C_{18}$ ou un groupe aryle en $C_6$-$C_{15}$.

3. Composés de formule:

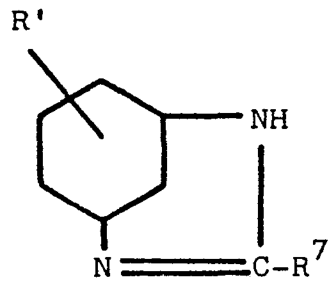

(1a)

où R' représente un groupe alkyle en $C_1$-$C_4$ ou un atome d'hydrogène, et $R^7$ a la signification indiquée dans la revendication 2.

4. Composés selon les revendications 2 et 3, caractérisés en ce que R' représente un groupe méthyle.

5. Procédé de préparation des composés selon les revendications 2-4, caractérisé en ce qu'on fait réagir un acide carboxylique de formule $R^7$-COOH ou un de ses dérivés réactifs avec 1 à 2,5 moles (rapporté à 1 mole de l'acide carboxylique utilisé ou de son dérivé), d'une diamine de formule:

$H_2N$-$C(R^1R^2)$-$C(R^3R^4)$-$C(R^5R^6)$ $NH_2$

à 150-280° C.